# EUROPEAN PATENT APPLICATION

(11) **EP 4 273 868 A1**
(43) Date of publication of application: **08.11.2023**
(21) Application number: 21915675.9
(22) Date of filing: 23.12.2021
(51) Int. Cl.: G16B 50/30, G16B 99/00, G16B 40/20, G06Q 10/10

(54) **SYSTEM FOR AUTOMATICALLY ISSUING PERIODICALLY UPDATED GENETIC MUTATION TEST RESULT REPORT**

(30) Priority: 29.12.2020 KR 20200186595
(71) Applicant: Pitter Petter Inc., Jeju-si, Jeju-do 63278 (KR)
(72) Inventor: SON, Ji Won, Seoul 05319 (KR); CHOI, Go Un, Seoul 07215 (KR)
(74) Representative: AWA Sweden AB
(86) International application number: PCT/KR2021/019723
(87) International publication number: WO 2022/145877

(57) **Abstract**

The present invention relates to a system for automatically issuing a periodically updated genetic mutation test result report, and to a computer-readable recording medium in which a program for executing the system for automatically issuing a periodically updated genetic mutation rest result is recorded.

## Description

### [Technical Field]

The present invention relates to a system for automatically issuing a periodically updated genetic variation test result report.

### [Background Art]

In the cells of living things, there is a genetic material called deoxyribonucleic acid (DNA). This material contains genetic information for constituting a living thing, and the living things continue to transmit their genetic information to future generations by passing on their DNA to their offspring. Such DNA has four types of bases and is a macromolecule having a double helix structure containing three billion pairs of bases. The genetic information of an individual entity differs depending on the composition of a nucleotide sequence, which ultimately determines characteristics of the entity. Also, all cells in one entity have the same number of chromosomes and genetic information. Accordingly, it is possible to know entire genetic information by analyzing only one cell.

Since the completion of the human genome project in 2003, which attempted to find the entire human genome sequence, genetic information analysis technology has developed worldwide, promoting genetic research and genetic testing related to diseases in humans and various living things. Accordingly, the cost of genetic analysis has gradually decreased, accessibility to genetic testing has increased, and we have entered the era of precision medicine and individual/entity-customized medicine for efficient health management.

In genetic testing, genetic information of an entity is analyzed to predict the risk of a genetic disease developing and help with customized prevention strategy. Further, in genetic testing, a diagnosis of a genetic disease or sensitivity to drugs can be analyzed to help increase treatment effectiveness. In addition to improved health of each entity, social and national benefits can also be expected due to effects such as medical cost reduction and the like.

When genetic analysis is requested, a process related to receiving entity information according to a requester's requirements and a type of suspected disease, prior consultation, test analysis, and test result report and related information collection and genetic analysis are performed. However, data accuracy is low, and provided results are insufficient, and thus improvements are required.

(Patent document 1) Korean Patent Application Publication No. 10-2011-0035663 (April, 6, 2011), Record Media for Genetic Data Counseling Service Program Using Personal Genetic Security Data.

### [Disclosure]

### [Technical Problem]

The present invention is directed to providing a system for automatically issuing a periodically updated genetic variation test result report which is automatically synchronized and updated with a genetic variation test result of a vertebrate and research results updated in real time through natural language processing.

According to the present invention, a web community space in which people can talk with other users having the same genetic variation and phenotype about not only information related to genetic variations and phenotypes of vertebrates but also disease symptoms, examination methods, other factors, care methods, related diseases, and precautions for breeding is provided, and thus it is possible to help understand genetic variations and phenotypes of vertebrates.

Also, according to the present invention, the above diseases, symptoms, medical practices, etc. are mapped and managed with international medical codes and can be used later as feedback data for vertebrate genetic variation research. In particular, this can contribute to genetic variation research projects which are smaller in scale than those of humans such as companion animal genetic variation research projects.

### [Technical Solution]

One aspect of the present invention provides a system for automatically issuing a periodically updated genetic variation test result report, the system including a) a disease/gene variation database build-up module configured to extract gene data, genetic variation site data, and phenotype data from information collected by an article database using an automatic named entity recognition (NER) tool, verify content of the extracted data, and then automatically load the data, b) a disease metadata build-up module configured to load data into an external phenotype metadata database and a phenotype data table to collect phenotype metadata related to a genotype of a specific animal species, c) a data mapping module configured to add a sentence to a report sentence data table according to each report on the basis of a phenotype data table and genetic variation data table and map an identifier (ID) of an image element to be used to each report in order to automate report generation, and d) a result provision module configured to receive a genetic variation information (variant call format (VCF)) file of a customer's animal as an input value to extract a genetic variation, automatically generate a phenotype-specific report according to an actual variation of the animal, and provide the phenotype-specific report so that the customer may download the phenotype-specific report.

Another aspect of the present invention provides a computer-readable recording medium on which a program for running the system for automatically issuing a periodically updated genetic variation test result report is recorded.

### [Advantageous Effects]

A system for automatically issuing a periodically updated genetic variation test result report according to the present invention automatically generates a report reflecting the latest research result according to a genetic variation and phenotype information of a vertebrate, periodically updates the report, and thus can provide rapid and accurate genetic variation test results of the vertebrate.

### [Description of Drawings]

FIG. 1 is a simulation diagram illustrating an embodiment of a) a disease/gene variation database build-up module in a system for automatically issuing a periodically updated genetic variation test result report according to the present invention.
FIG. 2 is a simulation diagram illustrating another embodiment of a) a disease/gene variation database build-up module in the system for automatically issuing a periodically updated genetic variation test result report according to the present invention.
FIG. 3 is a simulation diagram illustrating an embodiment of b) a disease metadata build-up module in the system for automatically issuing a periodically updated genetic variation test result report according to the present invention.
FIG. 4 is a simulation diagram illustrating an embodiment of c) a data mapping module in the system for automatically issuing a periodically updated genetic variation test result report according to the present invention.
FIG. 5 is a simulation diagram illustrating an embodiment of d) a result provision module in the system for automatically issuing a periodically updated genetic variation test result report according to the present invention.
FIGS. 6 to 9 are results showing webpages according to the embodiment of d) the result provision module in the system for automatically issuing a periodically updated genetic variation test result report according to the present invention.

### [Best Mode of the Invention]

Hereinafter, the present invention will be described in further detail.

Terms or words used in this specification and claims should not be construed as being limited to their usual or dictionary meanings. Based on the principle that the inventor can appropriately define the concept of a term to explain his or her invention in the best way, terms or words should be interpreted with meanings and concepts consistent with the technical spirit of the present invention.

Terminology used herein is only used to describe a specific embodiment and is not intended to limit the present invention. Singular forms include plural forms unless the context clearly indicates otherwise. In the present invention, it is to be understood that the terms "include," "have," etc. indicate the presence of features, numerals, steps, operations, components, parts, or combinations thereof described herein and does not preclude the presence or addition of one or more other features, numerals, steps, operations, components, parts, or combinations thereof.

The term "article metadata" used herein means metadata of an article. This may include the title of the article, a journal in which the article was published, an author, the year of publication, a digital object identifier (DOI), a PubMed identifier (ID), Medical Subject Headings (MeSH) terms mapped to the article, etc.

The term "phenotype metadata" used herein means metadata of a phenotype. This may include the name of the phenotype, a National Center for Biotechnology Information (NCBI) taxonomy ID, whether the phenotype corresponds to a disease/characteristic, an Online Mendelian Inheritance in Animals (OMIA) database phenotype ID, a genetic form, a MeSH terms ID corresponding to the phenotype, etc.

The term "genetic variation metadata" used herein means metadata of a genetic variation. This may include the NCBI ID of a gene with the corresponding genetic variation, a gene name designated by the NCBI of the gene, a chromosome number, a Vertebrate Gene Nomenclature Committee (VGNC) ID, whether the genetic variation is a deleterious mutation, a reference sequence (refseq) version, a database single nucleotide variations (dbSNP) ID, the site of the genetic variation, the site of a protein mutation, an OMIA database phenotype ID, an OMIA database genetic variation ID, etc.

The term "report sentence data" used herein means a sentence to be actually written in a report. A "report" of this specification is output with different content and in different forms depending on customer, vertebrate or companion animal, and phenotype and is automatically generated by an automated system. For this automation, the report is stored with a unique ID in a sentence data table in a database. Report sentence data may include a sentence ID, a sentence type, sentence content, etc.

The term "specific animal" used herein may mean an animal corresponding to a vertebrate, which may include humans.

Compared to a database of vertebrates, a database of humans has plenty of information which is well organized, and shows genetic similarities with other vertebrates. Accordingly, research on humans can be referred to in research on vertebrates. For example, Systematized Nomenclature Of Medicine-Clinical Terms (SNOMED-CT) is a medical terminology system code which includes diseases and widely defines other medical treatments, and Online Mendelian Inheritance in Man (OMIM) is a database in which extensive information related to phenotype-genotype correlation is systematically organized. OMIM includes genetic functions, biochemical and molecular biological information on phenotypes, related animal models, genome and protein information, clinically relevant information, etc.

The term "phenotype" used herein refers to an expressive characteristic resulting from a genotype. This means not only a disease or symptom but also expressed characteristics such as eye color, fur color, etc.

The term "module" used herein means one unit for processing a specific function or operation. This may be implemented as hardware, software, or a combination of hardware and software.

A system for automatically issuing a periodically updated genetic variation test result report according to the present invention includes:
a) a disease/gene variation database build-up module which extracts gene data, genetic variation site data, and phenotype data from information collected by an article database using an automatic named entity recognition (NER) tool, verifies content of the extracted data, and then automatically loads the data;
b) a disease metadata build-up module which loads data into an external phenotype metadata database and a phenotype data table to collect phenotype metadata related to a genotype of a specific animal species;
c) a data mapping module which adds a sentence to a report sentence data table according to each report on the basis of a phenotype data table and a genetic variation data table and maps an identifier (ID) of an image element to be used to each report in order to automate report generation; and
d) a result provision module which receives a genetic variation information (variant call format (VCF)) file of a customer's animal as an input value to extract a genetic variation, automatically generates a phenotype-specific report according to an actual variation of the animal, and provides the phenotype-specific report so that the customer may download the phenotype-specific report.

In the system for automatically issuing a periodically updated genetic variation test result report according to the present invention, a) the disease/gene variation database build-up module may include:
a-1) a module which prepares a search word for finding a genetic variation-disease relationship of the specific animal species and a unique structural search word ID (a MeSH terms ID) through an NCBI taxonomy ID of the specific animal species and a target of search;
a-2) a module which requests the module of a-1) to search a keyword list for the specific animal species using an article search application programming interface (API) of an external article database and extracts an extraction column selected from the group consisting of a PubMed ID, a DOI, an article title, an article abstract, and MeSH terms as result data;
a-3) a module which determines whether there is an overlapping entity in an existing article metadata table on the basis of the extraction column;
a-4) a module which uses an article abstract of each article as input data for PubTator which is an automatic bio-entity recognition tool;
a-5) a module which separately extracts only gene data, genetic variation site data, and phenotype data of a bio-entity extracted as result data of PubTator;
a-6) a module which extracts gene data, genetic variation site data, and phenotype data like the module of a-2);
a-7) a module which automatically extracts "gene-genetic variation-phenotype" relationships using results of the module of a-4) and the module of a-5);
a-8) a module which determines whether an article is related to a target animal species of an article to be originally extracted and whether the article describes the correlation between a genotype variation and phenotype of the animal species with reference to the article title and the article abstract among results of the module of a-2);
a-9) a module which checks, when results of the module of a-8) indicate true, a correctly extracted relationship by comparing article titles and article abstracts among results of the module of a-7) with results of the module of a-2) and determines, when the results of the module of a-8) indicate false, the article as an incorrectly extracted article and provides feedback for improving performance of a) the extraction module of the module of a-2), that is, feedback of MeSH terms which may be helpful for extraction, and a reason that the article is not a related article,
a-10) a module which checks a correctly extracted relationship among the extracted "gene-genetic variation-phenotype" relationships when the results of the module of a-8) indicate true in the module of a-9);
a-11) a module which determines whether there is a correctly extracted relationship from the result of the module of a-10) and provides, when there is a correctly extracted relationship, feedback of what kind of relationship the correctly extracted relationship is to improve the module of a-5);
a-12) a module which extracts "gene-genetic variation-phenotype" relationships of a module on the basis of various types of information such as a result of natural language analysis performed by a program developer on a sentence from which the relationship is extracted on the basis of a feedback result of the module of a-11), a result of the module of a-5), the distance between the strings of bio-entity names, etc.;
a-13) a module which checks and records the number of test object entities (experimental group/control group) in the article;
a-14) a module which displays low reliability for the article when the article is determined to have low reliability due to the excessively small number of test object entities from the result of the module of a-13) and then does not reflect the gene-genetic variation-phenotype relationships of the article having low reliability on gene test results or displays that the article has low reliability; and
a-15) a module which adds metadata, genetic variation metadata, and a phenotype name (the name of a phenotype) of the article to the database when the reliability is sufficiently high in the result of the module of a-13).

In the system for automatically issuing a periodically updated genetic variation test result report according to the present invention, b) the disease metadata build-up module may include:
b-1) a module which uses a "phenotype" ID and a species ID in "gene-genetic variation-phenotype" relationships existing in the article metadata table as input values to acquire a phenotype name of a corresponding data table C and an NCBI MeSH terms ID;
b-2) a module which acquires the search results of the module of b-1) using an API of the external reference phenotype metadata database;
b-3) a module which writes description, diseases, and symptoms of the phenotype of the animal species on the basis of search results of the module of b-2);
b-4) a module which maps the NCBI MeSH ID to the diseases and symptoms of the phenotype written by the module of b-3); and
b-5) a module which updates a phenotype metadata table with results of the module of b-1) to the module of b-4) and maintains up-to-date data by periodically repeating the process.

In the system for automatically issuing a periodically updated genetic variation test result report according to the present invention, c) the data mapping module may include:
c-1) a module which writes a sentence to be actually included in a report on the basis of the phenotype data table and the genetic variation data table;
c-2) a module which adds content examined by the module of c-1) to the sentence data table;
c-3) a module which maps a corresponding sentence ID to a unique report ID of a specific report (a specific animal species-a specific disease-a specific genetic variation);
c-4) a module which stores, in the sentence data table, an absolute path of an image to be used in the report in a server;
c-5) a module which maps a corresponding image ID to the unique report ID of the specific report (a specific animal species-a specific disease-a specific genetic variation); and
c-6) a module which updates the report sentence data table with results of the module of c-2) to the module of c-5) and maintains up-to-date data by periodically repeating the process.

In the system for automatically issuing a periodically updated genetic variation test result report according to the present invention, d) the result provision module may include:
d-1) a module which acquires the genetic variation information (VCF) file of the customer's animal;
d-2) a module which checks a genetic variation of the customer's animal through the genetic variation data table of the animal species;
d-3) a module which records the genetic variation of the customer's animal in an animal data table of customers;
d-4) a module which loads a sentence to be displayed in a report from the sentence data table on the basis of a result of the module of d-3) and a "unique ID of the genetic variation;"
d-5) a module which loads an image to be displayed in the report from the sentence data table on the basis of the result of the module of d-3) and the "unique ID of the genetic variation;" and
d-6) a module which generates webpages of phenotype-specific reports through report automation code written in a programming language on the basis of results of the module of d-4) and the module of d-5).

In the system for automatically issuing a periodically updated genetic variation test result report according to the present invention, the module of a) to the module of c) may be periodically repeated.

In the system for automatically issuing a periodically updated genetic variation test result report according to the present invention, all possible relationships between "gene," "genetic variation," and "phenotype" entities are extracted, and then priority rankings are given to the extracted relationships by giving weights to the relationships on the basis of a specific condition so that the "gene-genetic variation-phenotype" relationships of the module of a-7) may be automatically extracted.

In the system for automatically issuing a periodically updated genetic variation test result report according to the present invention, the search results of the module of b-2) may be any one or more of general description, related diseases, and related symptoms of a specific phenotype.

In the system for automatically issuing a periodically updated genetic variation test result report according to the present invention, data recorded by the module of d-3) may be any one or more selected from the group consisting of the unique ID of the genetic variation (from a genetic variation table), information on mutated alleles, and the number of mutated alleles.

The present invention provides a computer-readable recording medium on which a program for running the system for automatically issuing a periodically updated genetic variation test result report is recorded.

### [Modes of the Invention]

A system for automatically issuing a periodically updated genetic variation test result report according to the present invention includes a) a disease/gene variation database build-up module.

In the present invention, the disease/gene variation database build-up module extracts gene data, genetic variation site data, and phenotype data from information collected by an article database using an automatic named entity recognition (NER) tool, verifies content of the extracted data, and then automatically loads the data.

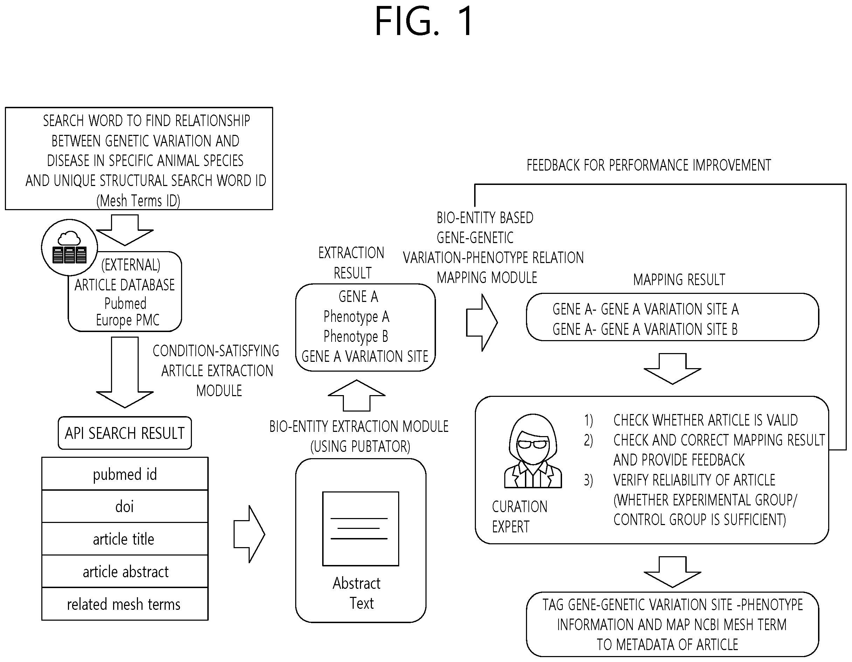

The disease/gene variation database build-up module may be illustrated through FIG. 1 and will be described in detail below.

In the present invention, a) the disease/gene variation database build-up module may be implemented through a-1) a module to a-15) a module as described below.

The article database may be an external article which discloses correlation between genotypes and phenotypes of a specific animal species.

First, the module of a-1) may prepare a search word for finding a genetic variation-disease relationship of the specific animal species and a unique structural search word ID (a Medical Subject Headings (MeSH) terms ID) through an NCBI taxonomy ID (a unique number given to the animal species) of the specific animal species and a target of search (NCBI MeSH terms). For example, 9685[uid] AND "mutation"[MeSH Terms]" AND "Disease Category"[MeSH terms]" is prepared.

Subsequently, the module of a-2) may request the module of a-1) to search a keyword list for the specific animal species using an article search application programming interface (API) of the external article database (e.g., PubMed or Europe PubMed Central (PMC)) and extract result data. Here, a PubMed ID, a digital object identifier (DOI), an article title, an article abstract, MeSH terms, etc. may be used as an extraction column.

Subsequently, the module of a-3) may determine whether there is an overlapping entity in an existing article metadata table on the basis of the PubMed ID and the DOI.

Subsequently, the module of a-4) may use an article abstract of each article as input data for PubTator which is an automatic bio-entity recognition tool.

Subsequently, the module of a-5) may separately extract only gene data, genetic variation site data, and phenotype data of a bio-entity extracted as result data of PubTator. Here, the phenotype data is data of diseases, symptoms, etc.

Subsequently, the module of a-6) may extract gene data, genetic variation site data, and phenotype data like "MeSH terms" of the module of a-2).

Subsequently, the module of a-7) may automatically extract "gene-genetic variation-phenotype" relationships using results of the module of a-4) and the module of a-5). All possible relationships between "gene," "genetic variation," and "phenotype" entities are extracted, and then priority rankings are given to the extracted relationships by giving weights to the relationships on the basis of a specific condition so that the "gene-genetic variation-phenotype" relationships may be automatically extracted.

Subsequently, the module of a-8) may enable a curation expert to determine whether an article is related to a target animal species of an article to be originally extracted and whether the article describes the correlation between a genotype variation and phenotype of the animal species with reference to the article title and the article abstract among results of the module of a-2).

Subsequently, the module of a-9) may check, when results of the module of a-8) indicate true, a correctly extracted relationship by comparing article titles and article abstracts among results of the module of a-7) with results of the module of a-2) and determine, when the results of the module of a-8) indicate false, the article as an incorrectly extracted article and provide feedback for improving performance of a) the extraction module of the module of a-2), that is, feedback of MeSH terms which may be helpful for extraction, and a reason that the article is not a related article.

Subsequently, the module of a-10) may check a correctly extracted relationship among the extracted "gene-genetic variation-phenotype" relationships when the results of the module of a-8) indicate true in the module of a-9). Here, when there is no correctly extracted "gene-genetic variation-phenotype" relationship, the module of a-10) may write a gene-genetic variation-phenotype relationship.

Subsequently, the module of a-11) may determine whether there is a correctly extracted relationship from the result of the module of a-10) and provide, when there is a correctly extracted relationship, feedback of what kind of relationship the correctly extracted relationship is to improve the module of a-5).

Subsequently, the module of a-12) may improve a module to extract "gene-genetic variation-phenotype" relationships of the module more accurately on the basis of various types of information such as a result of natural language analysis performed by a program developer on a sentence from which the relationship is extracted on the basis of a feedback result of the module of a-11), a result of the module of a-5), the distance between the strings of bio-entity names, etc.

Subsequently, the module of a-13) may check and record the number of test object entities (experimental group/control group) in the article.

Subsequently, the module of a-14) may display low reliability for the article when the article is determined to have low reliability due to the excessively small number of test object entities in the result of the module of a-13) and then may not reflect the gene-genetic variation-phenotype relationships of the article having low reliability in gene test results or may reflect it in the gene test results but display that the article has low reliability.

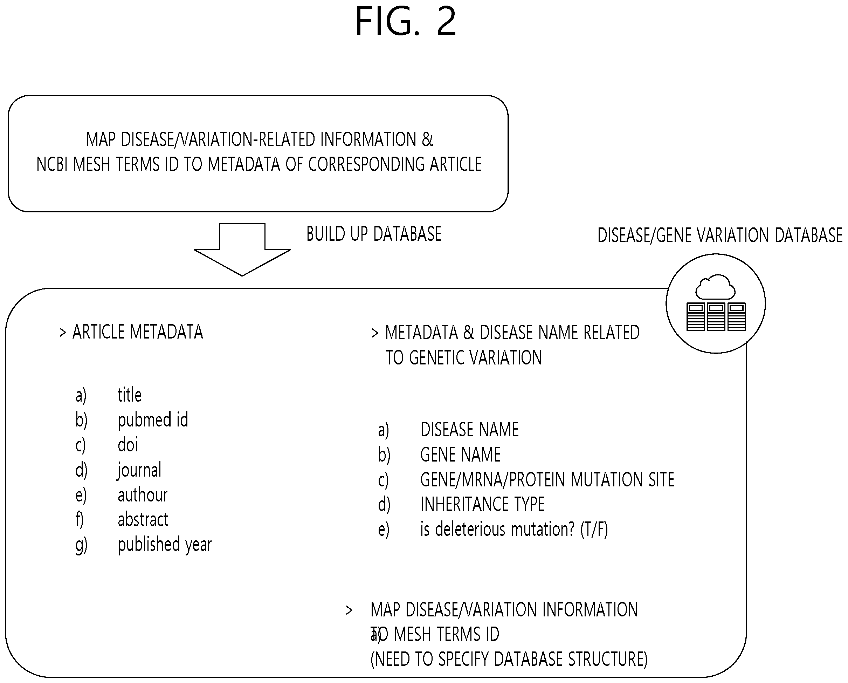

Subsequently, as shown in FIG. 2, the module of a-15) may add metadata, genetic variation metadata, and a phenotype name (the name of a phenotype) of the article to the database when the reliability is sufficiently high in the result of the module of a-13). The article metadata is a title, a PubMed ID, a DOI, a journal title, an author, an abstract, a publishing year, etc. The genetic variation metadata is a phenotype, a gene, a variant (genetic variation), an inheritance type, a deleterious mutation (T/F), reliability, a species, etc. The phenotype ID, the gene ID, and the variant ID therein may be assigned an NCBI MeSH terms ID for a corresponding entity in addition to a unique ID given by a company database. With results of the module a which is the disease/gene variation database build-up module, the article metadata table and a genetic variation metadata table are updated, and the corresponding process is periodically repeated to maintain up-to-date data.

The system for automatically issuing a periodically updated genetic variation test result report according to the present invention includes b) a disease metadata database build-up module.
b) The disease metadata database build-up module may load data into an external phenotype metadata database and a phenotype data table to collect phenotype metadata related to genotypes of a specific animal species. In the present invention, a specific animal may be a vertebrate, such as human, but is preferably a companion animal.

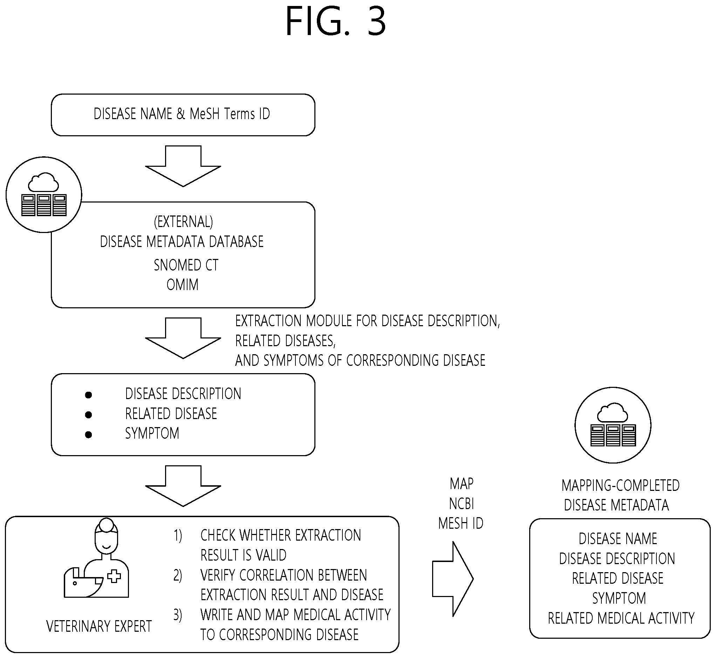

The disease/gene variation database build-up module may be illustrated through FIG. 3 and will be described in detail below.

In the present invention, b) the disease metadata database build-up module may be implemented through b-1) a module to b-5) a module as described below.

First, the module of b-1) may uses a "phenotype" ID and a species ID in "gene-genetic variation-phenotype" relationships existing in the article metadata table as input values to acquire a phenotype name of a corresponding data table C and an NCBI MeSH terms ID.

Subsequently, the module of b-2) may acquire the search results of the module of b-1) using an API of the external reference phenotype metadata database (Systematized Nomenclature Of Medicine-Clinical Terms (SNOMED CT) 2 and Online Mendelian Inheritance in Man (OMIM) 3). The search results may be general description, related diseases, and related symptoms of a specific phenotype. Also, the external reference phenotype metadata database is based on humans and thus may be used only for reference.

Subsequently, the module of b-3) may write description, diseases, and symptoms of the phenotype of the animal species on the basis of the search results of the module of b-2). Here, when necessary, a veterinary expert may write medical activities for the phenotype, and the medical activities may be a test for diagnosing the phenotype, treatment which may be provided to an animal having the phenotype, etc.

Subsequently, the module of b-4) may map the NCBI MeSH ID to the diseases and symptoms of the module of b-3). Also, the module of b-4) may map the NCBI MeSH ID to the medical activities of the module of b-3).

Subsequently, the module of b-5) may update a phenotype metadata table with the results of the module of b-1) to the module of b-4) and maintain up-to-date data by periodically repeating the process.

The system for automatically issuing a periodically updated genetic variation test result report according to the present invention includes c) a data mapping module.

To automate report generation, c) the data mapping module may add a sentence to a report sentence data table according to each report on the basis of the phenotype data table and a genetic variation data table and map an ID of an image element to be used to each report.

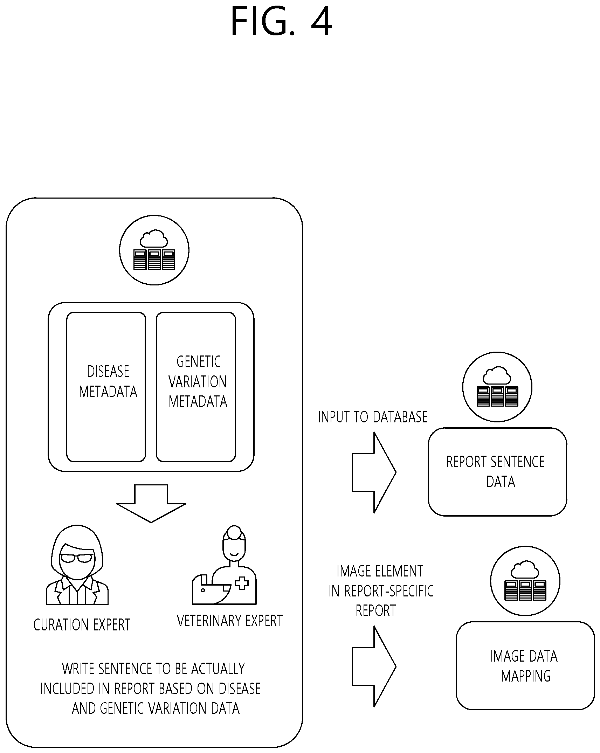

The data mapping module may be illustrated through FIG. 4 and will be described in detail below.

In the present invention, c) the data mapping module may be implemented through c-1) a module to c-6) a module as described below.

In c) the data mapping module, each report may be generated according to "a specific animal-a specific disease-a specific genetic variation."

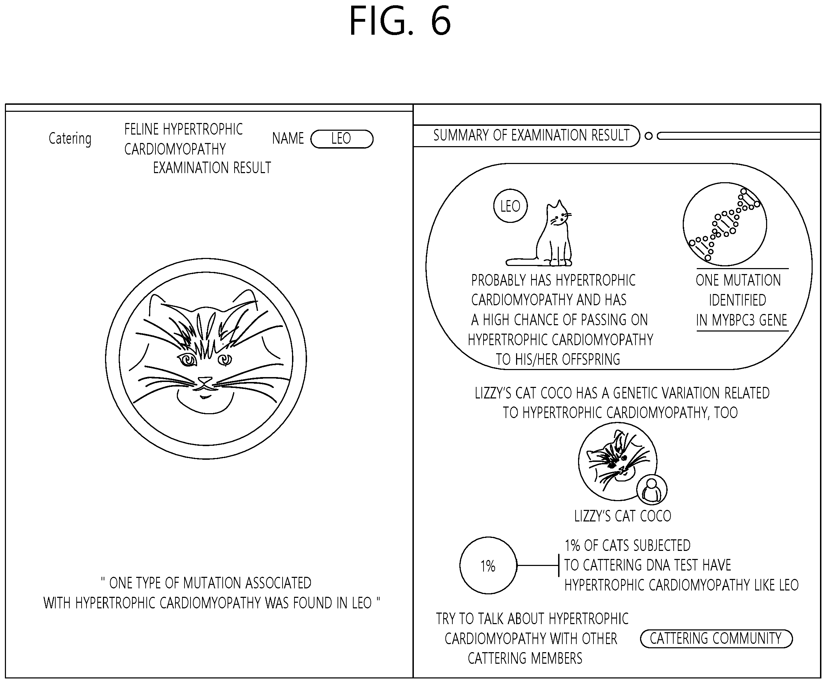

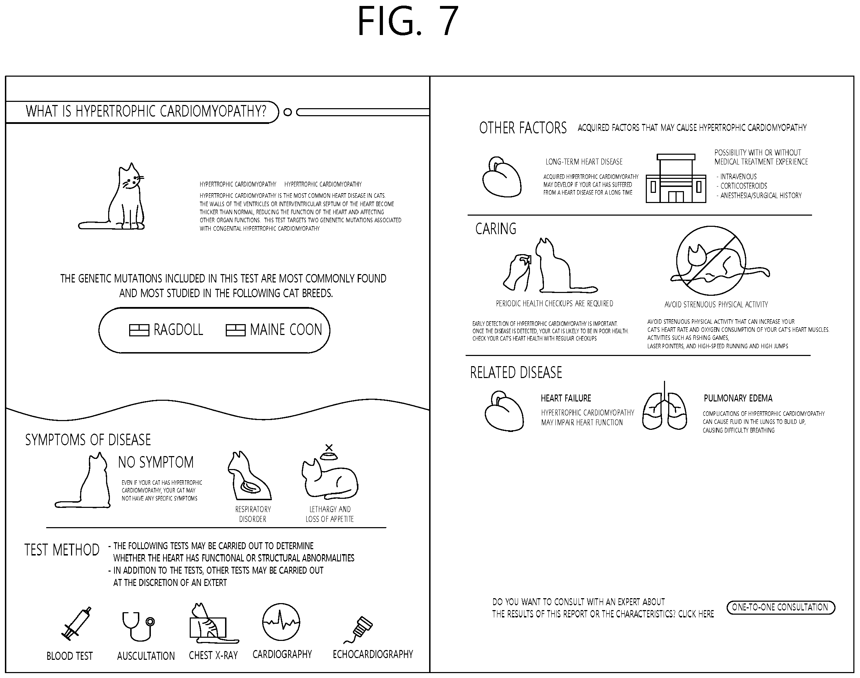

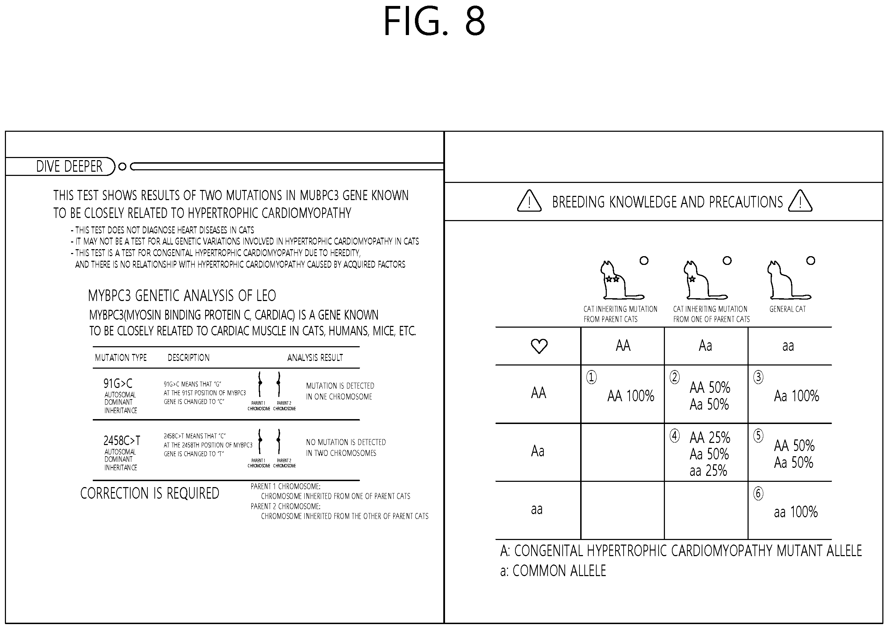

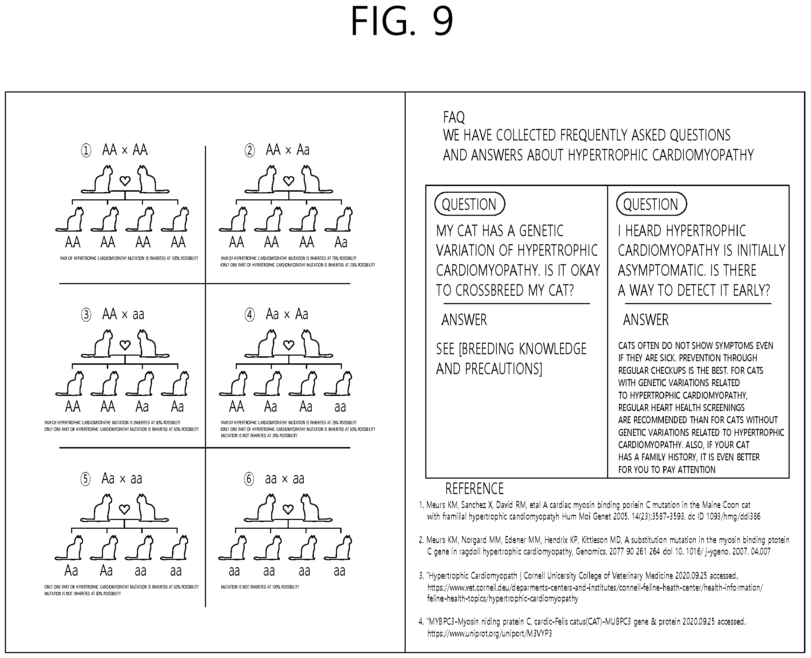

First, the module of c-1) may write a sentence to be actually included in a report on the basis of the phenotype data table and the genetic variation data table. The sentence to be included in the report may be written according to a reference and method for writing a sentence as shown in Table 1 below, and the actual sentence may be written as shown in FIGS. 6 to 9 which will be described below. Sentences of Table 1 below may be written by a curation expert and a veterinary expert who examine the content, but are not necessarily limited to these cases.

**[Table 1]**

| For veterin arian | Unique number | Area division | Fixed column | Data name | Data description | Data volume | Data characteristic |
|---|---|---|---|---|---|---|---|
| | 1 | upper | Y | disease or character istic entity name | name of disease or characteristic | one word | most frequently used name in common use |
| | 2 | upper | Y | disease or character istic synonym | synonym of disease or characteristic | | |
| | 3 | upper | Y | disease or character istic entity descripti on | brief description of disease or characteristic | three or four rows or less | understandab le and clear expression |
| | 4 | upper | | summar y of test result | whether animal has disease or characteristic | one sentence | including name of companion animal |
| | 5 | upper | | brief descripti on of test result | sentence describing details of 3 | two sentence s or less | effect of meaning of 3 |
| | 6 | upper | Y | purpose of correspo nding report | only mention that test has been conducted for variation related to corresponding disease | | only mention matters based on facts |
| | 7 | upper | Y | limit of correspo nding report | whether current health condition is related to disease diagnosis and notable points | two rows or less per item | whether correspondin g disease or characteristic can be current health condition or disease diagnosis (generally not), and other symptoms to watch out for that occur with correspondin g disease |
| | 8 | upper | Y | relation to breed | breed (same concept as human race) related to corresponding variation which is target of test | only mention each item | brief description mainly with facts |
| | 9 | middle_upper | | brief descripti on of genetic variation | simply mention whether variation is detected | one row or less per item | |
| T | 10 | middle _upper | Y | prevalen ce of correspo nding disease | prevalence of correspondin g disease | four rows or less | |
| T | 11 | middle _upper | Y | other factors which may influenc e correspo nding disease or characteristic | age, environment, sex, family history, daily habits, etc. | three rows or less per item | |
| T | 12 | middle _upper | Y | general descripti on related to disease character istic | description of disease characteristic | three rows or less | ^{∗}characteristi c *related symptom *related disease *care method *related test |
| T | 13 | middle _upper | Y | sympto ms related to disease character istic | symptom and syndrome related to correspondin g disease characteristic | one row or less per item | |
| T | 14 | middle _upper | Y | other diseases related to disease character istic | other diseases related to corresponding disease characteristic | | |
| T | 15 | middle_upper | Y | test related to disease character istic | test list related to correspondin g disease characteristic | three rows or less per item | |
| T | 16 | middle _upper | Y | care method for disease character istic | care method related to corresponding disease characteristic | | |
| T | 17 | middle _upper | Y | recomm end consultat ion with veterinar ian for disease character istic | recommend conversation with veterinarian about correspondin g report things worth knowing for conversation with veterinarian (linked to 12) | one row or less per item | |
| | 19 | middle_bottom | | breeding guide for correspo nding compani on animal accordin g to test result | comment on breeding of correspondin g companion animal according to test result | two rows or less | |
| | 18 | middle _botto m | Y | breeding knowled ge accordin g to test result | breeding information according to whether correspondin g companion animal has correspondin g variation | four rows or less | |
| | 20 | middle_bottom | | comparis on with family, friends, and other members | comparison with family, friends, etc. of corresponding companion animal | two rows or less per guide phrase | |
| | 21 | middle_bottom | | comparis on with PitterPet ter members | prevalence comparison with data of members in Cattering/Do gmar (n% higher or lower than members) | needs expansio n | |
| | 22 | middle _botto m | Y | induce commun ication through message board | message board for communicati on with other partners of other companion animals with correspondin g disease or characteristic | one row | |
| | 23 | middle _bottom | Y | question s and answers | link to 20 | one row | |
| T | 24 | bottom | Y | descripti on of correspo nding gene | scientific description of correspondin g gene | four rows or less | |
| T | 25 | bottom | Y | detailed descripti on of correspo nding genetic variation | IDs for other expert database for reference (reference single-nucleotide polymorphis m (RS) ID, NCBI ID, etc.), and reference | four rows or less of descriptive explanation of corresponding genetic variation | |
| | 26 | bottom | Y | referenc es and data | bibliography and data list | depending on number of items | |
| | 27 | bottom | Y | update log | content of changes and the like caused by academic updates after initial date of preparation of correspondin g report | depending on number of items | |
| | | 28 | bottom | Y | frequentl y asked question s | frequently asked questions and answers about correspondin g disease or characteristic | depending on number of items |

Subsequently, the module of c-2) may add content examined by the module of c-1) to the sentence data table as shown in Table 1 above. The sentence data has a unique ID and may be used repeatedly in multiple reports.

Subsequently, the module of c-3) may map a corresponding sentence ID to a unique report ID of a specific report (a specific animal species-a specific disease-a specific genetic variation).

Subsequently, the module of c-4) may store, in the sentence data table, an absolute path of an image to be used in the report in a server. As a result, image data has a unique ID and may be used repeatedly in multiple reports.

Subsequently, the module of c-5) may map a corresponding image ID to the unique report ID of the specific report (the specific animal species-the specific disease-the specific genetic variation).

Subsequently, the module of c-6) may update the report sentence data table with results of the module of c-2) to the module of c-5) and maintain up-to-date data by periodically repeating the process.

The system for automatically issuing a periodically updated genetic variation test result report according to the present invention includes d) a result provision module.
d) The result provision module may receive a genetic variation information (VCF) file of a customer's animal as an input value to extract a genetic variation, automatically generate a phenotype-specific report according to an actual variation of the animal, and provide the phenotype-specific report so that the customer may download the phenotype-specific report.

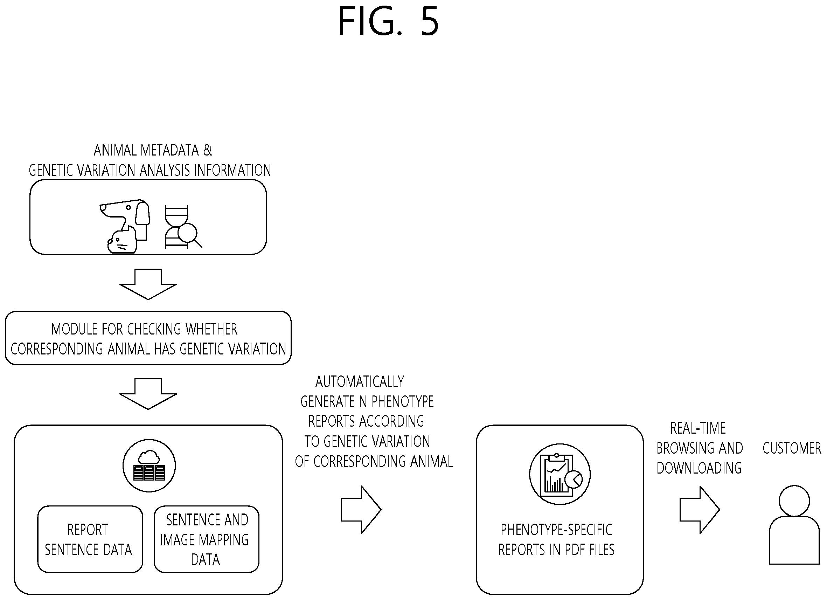

The disease/gene variation database build-up module may be illustrated through FIG. 5 and will be described in detail below.

In the present invention, d) the result provision module may be implemented through d-1) a module to d-6) a module as described below.

First, the module of d-1) may acquire the genetic variation information (VCF) file of the customer's animal. VCF is the format of a text file used in bioinformatics for storing gene sequence variations.

Subsequently, the module of d-2) may check a genetic variation of the customer's animal through the genetic variation data table of the animal species.

Subsequently, the module of d-3) may record the genetic variation of the customer's animal in an animal data table of customers. Here, the recorded data may be "the unique ID of a corresponding genetic variation" (from a genetic variation table), information on mutated alleles, and the number of mutated alleles. Also, the recorded data may be the species of the customer's animal, the name of the animal, the animal's sex, whether the animal has been neutered, the animal's breed, the animal's birthdate, etc.

Subsequently, the module of d-4) may load a sentence to be displayed in a report from the sentence data table on the basis of a result of the module of d-3) and the "unique ID of the genetic variation."

Subsequently, the module of d-5) may load an image to be displayed in the report from the sentence data table on the basis of the result of the module of d-3) and the "unique ID of the genetic variation;" and

Subsequently, the module of d-6) may generate webpages of phenotype-specific reports through report automation code written in a programming language on the basis of results of the module of d-4) and the module of d-5). Here, the webpages may be downloaded in the form of a portable document format (PDF) file, and an example is shown in FIGS. 6 to 9.

In the system for automatically issuing a periodically updated genetic variation test result report according to the present invention, the module of a) to the module of c) may be periodically repeated so that a result report of the module of d-6) may reflect up-to-date research data.

Another aspect of the present invention provides a computer-readable recording medium on which a program for running the system for automatically issuing a periodically updated genetic variation test result report according to the present invention is recorded.

Those skilled in the technical field to which the present invention pertains should understand that the present invention can be implemented in other specific forms without changing the technical spirit or essential features thereof. Therefore, the embodiments described above should be construed as illustrative and not restrictive in all aspects.

The scope of the present invention is represented by the following claims rather than the above detailed description, and all modifications or alterations derived from the meaning and scope of the claims and equivalents thereof should be construed as falling within the scope of the present invention.

## Claims

1. A system for automatically issuing a periodically updated genetic variation test result report, the system comprising:
a) a disease/gene variation database build-up module configured to extract gene data, genetic variation site data, and phenotype data from information collected by an article database using an automatic named entity recognition (NER) tool, verify content of the extracted data, and then automatically load the data;
b) a disease metadata build-up module configured to load data into an external phenotype metadata database and a phenotype data table to collect phenotype metadata related to a genotype of a specific animal species;
c) a data mapping module configured to add a sentence to a report sentence data table according to each report on the basis of a phenotype data table and a genetic variation data table and map an identifier (ID) of an image element to be used to each report in order to automate report generation; and
d) a result provision module configured to receive a genetic variation information (variant call format (VCF)) file of a customer's animal as an input value to extract a genetic variation, automatically generate a phenotype-specific report according to an actual variation of the animal, and provide the phenotype-specific report so that the customer is able to download the phenotype-specific report.

2. The system of claim 1, wherein a) the disease/gene variation database build-up module comprises:
a-1) a module configured to prepare a search word for finding a genetic variation-disease relationship of the specific animal species and a unique structural search word ID (a Medical Subject Headings (MeSH) terms ID) through a National Center for Biotechnology Information (NCBI) taxonomy ID of the specific animal species and a target of search;
a-2) a module configured to request the module of a-1) to search a keyword list for the specific animal species using an article search application programming interface (API) of an external article database and extract an extraction column selected from the group consisting of a PubMed ID, a digital object identifier (DOI), an article title, an article abstract, and MeSH terms as result data;
a-3) a module configured to determine whether there is an overlapping entity in an existing article metadata table on the basis of the extraction column;
a-4) a module configured to use an article abstract of each article as input data for PubTator which is an automatic bio-entity recognition tool;
a-5) a module configured to separately extract only gene data, genetic variation site data, and phenotype data of a bio-entity extracted as result data of PubTator;
a-6) a module configured to extract gene data, genetic variation site data, and phenotype data like the module of a-2);
a-7) a module configured to automatically extract "gene-genetic variation-phenotype" relationships using results of the module of a-4) and the module of a-5);
a-8) a module configured to determine whether an article is related to a target animal species of an article to be originally extracted and whether the article describes correlation between a genotype variation and phenotype of the animal species with reference to the article title and the article abstract among results of the module of a-2);
a-9) a module configured to check, when results of the module of a-8) indicate true, a correctly extracted relationship by comparing article titles and article abstracts among results of the module of a-7) with results of the module of a-2) and determine, when the results of the module of a-8) indicate false, the article as an incorrectly extracted article and provide feedback for improving performance of a) the extraction module of the module of a-2), that is, feedback of MeSH terms which are helpful for extraction, and a reason that the article is not a related article.
a-10) a module configured to check a correctly extracted relationship among the extracted "gene-genetic variation-phenotype" relationships when the results of the module of a-8) indicate true in the module of a-9);
a-11) a module configured to determine whether there is a correctly extracted relationship as a result of the module of a-10) and provide, when there is a correctly extracted relationship, feedback of what kind of relationship the correctly extracted relationship is to improve the module of a-5);
a-12) a module configured to extract "gene-genetic variation-phenotype" relationships of a module on the basis of various types of information such as a result of natural language analysis performed by a program developer on a sentence from which the relationship is extracted on the basis of a feedback result of the module of a-11), a result of the module of a-5), a distance between strings of bio-entity names, etc.;
a-13) a module configured to check and record the number of test object entities (experimental group/control group) in the article;
a-14) a module configured to display low reliability for the article when the article is determined to have low reliability due to the excessively small number of test object entities in the result of the module of a-13) and then not reflect the gene-genetic variation-phenotype relationships of the article having low reliability in gene test results or display that the article has low reliability; and
a-15) a module configured to add metadata, genetic variation metadata, and a phenotype name (a name representing a phenotype) of the article to the database when the reliability is sufficiently high in the result of the module of a-13).

3. The system of claim 1, wherein b) the disease metadata build-up module comprises:
b-1) a module configured to use a "phenotype" ID and a species ID in "gene-genetic variation-phenotype" relationships existing in an article metadata table as input values to acquire a phenotype name of a corresponding data table C and a National Center for Biotechnology Information (NCBI) Medical Subject Headings (MeSH) terms ID;
b-2) a module configured to acquire search results of the module of b-1) using an application programming interface (API) of the external reference phenotype metadata database;
b-3) a module configured to write description, diseases, and symptoms of the phenotype of the animal species on the basis of search results of the module of b-2);
b-4) a module configured to map the NCBI MeSH ID to the diseases and symptoms of the phenotype written by the module of b-3); and
b-5) a module configured to update a phenotype metadata table with results of the module of b-1) to the module of b-4) and maintain up-to-date data by periodically repeating the process.

4. The system of claim 1, wherein c) the data mapping module comprises:
c-1) a module configured to write a sentence to be actually included in a report on the basis of the phenotype data table and the genetic variation data table;
c-2) a module configured to add content examined by the module of c-1) to the sentence data table;
c-3) a module configured to map a corresponding sentence ID to a unique report ID of a specific report (a specific animal species-a specific disease-a specific genetic variation);
c-4) a module configured to store, in the sentence data table, an absolute path of an image to be used in the report in a server;
c-5) a module configured to map a corresponding image ID to the unique report ID of the specific report (the specific animal species-the specific disease-the specific genetic variation); and
c-6) a module configured to update the report sentence data table with results of the module of c-2) to the module of c-5) and maintain up-to-date data by periodically repeating the process.

5. The system of claim 1, wherein d) the result provision module comprises:
d-1) a module configured to acquire the genetic variation information (VCF) file of the customer's animal;
d-2) a module configured to check a genetic variation of the customer's animal through the genetic variation data table of the animal species;
d-3) a module configured to record the genetic variation of the customer's animal in an animal data table of customers;
d-4) a module configured to load a sentence to be displayed in a report from the sentence data table on the basis of a result of the module of d-3) and a "unique ID of the genetic variation;"
d-5) a module configured to load an image to be displayed in the report from the sentence data table on the basis of the result of the module of d-3) and the "unique ID of the genetic variation;" and
d-6) a module configured to generate webpages of phenotype-specific reports through report automation code written in a programming language on the basis of results of the module of d-4) and the module of d-5).

6. The system of claim 1, wherein the module of a) to the module of c) are periodically repeated.

7. The system of claim 2, wherein all possible relationships between "gene," "genetic variation," and "phenotype" entities are extracted, and then priority rankings are given to the extracted relationships by giving weights to the relationships on the basis of a specific condition so that the "gene-genetic variation-phenotype" relationships of the module of a-7) are automatically extracted.

8. The system of claim 3, wherein the search results of the module of b-2) are any one or more of general description, related diseases, and related symptoms of a specific phenotype.

9. The system of any one of claim 5, wherein data recorded by the module of d-3) is any one or more selected from the group consisting of the unique ID of the genetic variation (from a genetic variation table), information on mutated alleles, and the number of mutated alleles.

10. A computer-readable recording medium on which a program for running the system for automatically issuing a periodically updated genetic variation test result report is recorded.
